# EUROPEAN PATENT SPECIFICATION

(11) **EP 1 202 629 B1**
(45) Date of publication and mention of the grant of the patent: **10.11.2004**
(21) Application number: 00949805.6
(22) Date of filing: 07.08.2000
(51) Int. Cl.: A01N 63/00, C12N 1/20, C12N 15/82, C12N 15/70, C12N 15/52, C12N 15/63, C07K 14/325

(54) **INSECT CONTROL SYSTEM**
SYSTEM ZUR INSEKTENBEKÄMPFUNG
SYSTEME PERMETTANT DE CONTROLER UNE POPULATION D'INSECTES

(30) Priority: 06.08.1999 GB 9918536; 06.08.1999 GB 9918540; 23.08.1999 US 150272 P
(43) Date of publication of application: 08.05.2002
(73) Proprietor: The Foundation for Research and Technology, Institute of Molecular Biology and Biotechnology, Heraklion 71110 Crete (GR)
(72) Inventor: Craig, Roger, Smallwood, Sandbach, Cheshire CW11 0XA (GB); Savakis, Charalambos, 711 10 Heraklion (GR)
(74) Representative: Maschio, Antonio, Dr.
(86) International application number: PCT/GB2000/003043
(87) International publication number: WO 2001/010220

(56) References cited:
- WO-A-96/01055
- DRABEK, J. ET AL: "Proinsecticides" PROG. PESTIC. BIOCHEM. TOXICOL. (1985), 5 (INSECTICIDES ), 35-86 , - 1985 pages 35-85, XP000972860 Switzerland
- PRESTWICH, G. D. ET AL: "Harnessing insect-specific enzymes to activate novel proinsecticides" PESTICIDE SYNTHESIS THROUGH RATIONAL APPROACHES, (1984) PP. 127-143. 6 FIG. 24 REF. PUBLISHER: AMERICAN CHEMICAL SOCIETY. WASHINGTON, DC, XP000972855 USA
- PFEIFER, TOM A. ET AL: "Future perspectives on insect pest management: engineering the pest" J. INVERTEBR. PATHOL. (1996), 67(2), 109-19 , XP000972851
- LOUKERIS, T G ET AL: "Gene Transfer into the Medfly, Ceratitis capitata, with a Drosophila hydei Transposable Element" SCIENCE, - 1995 pages 2002-2005, XP000941764 Washington DC(USA)
- COURTWRIGHT, J.B AND KUMARAN, K A: "A Genetic Engineering Methodology for Insect Pest Control" BELTSVILLE SYMP. AGRIC. RES., vol. 10, - 1986 pages 325-326, XP000945144 USA
- O'BROCHTA, D A ET AL: "Building the Better Bug" SCIENTIFIC AMERICAN, vol. 279, no. 6, pages 60-65, XP000941766

## Description

### FIELD OF THE INVENTION

The present invention relates to methods for controlling insect populations using genetic techniques.

### BACKGROUND OF THE INVENTION

Insects are responsible for widespread damage to crops world-wide with enormous concomitant economic consequences. To try to reduce insect-inflicted damage, resources have been devoted to the development and deployment of insecticides, which control insect populations by killing target insects. Although insecticides are in many cases effective, they are known to be toxic to life forms other than target insects, which has important environmental consequences. It would therefore be advantageous to develop inactive pro-insecticides which are converted into their active form predominantly in the target insect.

Attempts have been made to control insects by biological means. For example, methods currently employed to control the populations of certain members of the dipteran class include the release of sterile males.

For example, as set forth in US patent 5, 840, 865, the Mediterranean fruit-fly (Medfly) *Ceratitis capitata* is a major agricultural pest for many fruit species that is geographically widespread in tropical and temperate regions. The Medfly has been introduced relatively recently into the New World, and appears to be spreading rapidly, threatening fruit producing areas in North America (Carey, J. R., Science 253: 1369 (1991)). Since the mid 1970's, the sterile insect technique has been used for Medfly eradication and control. This method relies on the decrease in or collapse of fly populations following releases of large numbers of sterile insects over infested areas, and offers an environmentally attractive alternative to massive spraying with insecticides (Knipling, E. F., Science 130: 902 (1959)).

Although the use of sterile male insects slows Medfly population growth and may lead to its temporary collapse, it does not lead to destruction of female insects, which are responsible for crop damage. Moreover, since the sterile males do not reproduce, the method requires repeated releases of sterile males into the environment. Recent improvements include the potential elimination of transgenic female in mass rearing environments using inducible terminator genes under the control of the antibiotic tetracycline (Heinrich and Scott, PNAS 97:8229-8232, 2000).

There therefore remains a need for a control technique for Medfly and other insects and other pests which can selectively destroy female or male insects but which is environmentally more acceptable than the mass spraying of toxic insecticides and does not require the large scale use of antibiotics for improved sterile techniques.

Furthermore, many human and veterinary health issues are associated with the spread of disease by insects. Examples include mosquitoes, tse-tse flies and the common housefly. Control of insect populations which endanger human or animal health is thus also desirable.

### SUMMARY OF THE INVENTION

The present invention provides a method for controlling a population of target insects, comprising:
a) providing a gene comprising a coding sequence encoding one constituent of an enzyme/pro-drug system and a promoter capable of driving the coding sequence in the target insect in a sex-specific manner;
b) transforming at least part of the population of target insects with the gene, and allowing the transposable elements to spread within the target insect population; and
c) administering to the population of target insects the remaining constituent(s) of the enzyme/pro-drug system.

As used herein, a "population of target insects" refers to a group of insects, whether delimited along species or geographical lines, or both, which it is desired to be controlled. For example, a population of insects may refer to a given species of insect which infests a particular crop in a given geographical area. Alternatively, it may refer to all insects infesting any crop in a geographical area, or a given species without reference to any geographical limitation, or a population of insects which is responsible for a human or veterinary health problem, such as the spread of malaria. Target insects are the individual members of the population of insects.

"Insecticide" as used herein means any pesticide that is meant to control any target insect by targeting the insect cells. Insecticides which can be modified according to the invention to be pro-insecticides include but are not limited to imidacloprid and methamidophos.

"Pro-insecticide", "pro-drug" and "pro-pesticide" are used interchangeably and as used herein mean any substantially inactive or substantially non-toxic substance in the absence of a converting enzyme, or mixture comprising such substance that can be converted to active or toxic substance by the action of an enzyme. Pro-insecticides can be specifically designed for the purpose or preferably designed by chemically modifying existing insecticides using, for example, methylation or acetylation as taught herein. The term "substantially" as used herein means "pro-insecticide", "pro-drug" and "pro-pesticide", which is at least 50%; 60%; 70%; 80%, 90%; 95%; 98% and up to and including 100% inactive when compared to the active form.

"Enzyme" as used herein means a reaction catalyzing substance including but not limited to protein or polypeptide or a fragment of such protein or polypeptide. An "enzyme" as used herein catalyzes a reaction which converts a pro-insecticide or pro-drug to be an insecticide or drug, respectively. Examples of enzymes include but are not limited to oxidases, esterases, and amidases.

"Control" as used herein refers to the limitation, prevention or reduction of growth, i.e., by at least about 10% per generation, preferably at least about 50%, 80%, or even up to and including 100% of the insect population. Preferably, this is achieved by killing target insects. Advantageously, the population of insects is eliminated.

"Gene", as used herein, refers to a nucleic acid sequence, usually DNA, which encodes a polypeptide or protein and additionally comprises the nucleic acid sequences required to transcribe the coding sequence in a suitable host cell. The nucleotide sequence encoding the polypeptide or protein is referred to herein as a "coding sequence" and the sequences required for regulation of sequence transcription are referred to as "control sequence", such as "enhancer" or "promoter."

The coding sequence encodes one constituent of an enzyme/pro-drug system. The constituent may be any one or more parts of the system, as long as it is not itself sufficient to produce or transform itself into the active drug from the pro-drug. Thus, the constituent is preferably an enzyme or a fragment of an enzyme which is responsible for pro-drug activation. Alternatively, it may be the pro-drug itself. The remaining constituent(s) of the enzyme/pro-drug system are administered separately, for example by spraying, thus killing the target insects which express the coding sequence according to the invention.

A feature of the present invention is that the promoter used to drive transcription of the coding sequence is functionally active in a sex-specific manner. This means that the coding sequence is expressed substantially only in one sex of the target insects, be it male or female. As used herein, "substantially only" refers to a selectivity for a given sex in the ratio of at least about 70:30, 80:20, 90: 10 or preferably 100:0. The gene which is used to transform the insects is preferably used to transform male target insects. Male transformation is especially preferred when a promoter which is used to drive the coding sequence is functional only in female target insects. This allows the selective destruction of female insects, while maintaining a population of male insects which are able to pass on the gene comprising the coding sequence whilst remaining unaffected by the pro-drug. This approach is highly advantageous where the female is responsible for spreading damage or disease.

In cases where the male is responsible for spreading damage or disease, the technique may be reversed, such that a coding sequence only functional in male insects is spread by carrier females.

The invention furthermore provides an insect of a given sex, which insect has been transformed with a gene comprising a coding sequence encoding one constituent of an enzyme/pro-drug system and a regulatory region, preferably promoter, operably linked and capable of driving the coding sequence substantially only in insects of the opposite sex. After selection in mass rearing conditions the surviving insects can be optionally irradicated for the use of sterile insect techniques.

In a further aspect, the invention provides a vector which is capable of transforming a target insect cell, which vector comprises a gene comprising a coding sequence encoding one constituent of an enzyme/pro-drug system and a promoter capable of driving the coding sequence in target insects in a sex-specific manner.

### BRIEF DESCRIPTION OF THE FIGURES

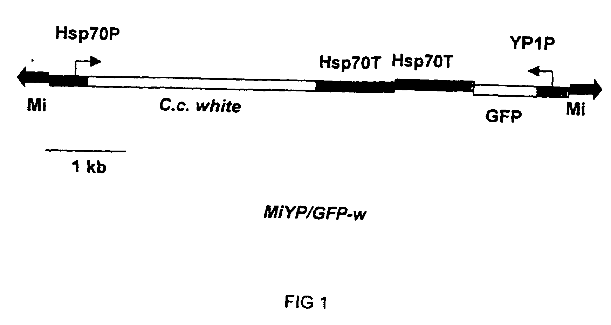

FIG. 1. Schematic representation of the YP/GFP transposon. Hsp70P is a promoter and Hsp70T is the terminator sequence of the *D. melanogaster* Hsp70 gene (Loukeris et al. 1995, Science 270, 2002-2005). *C. c. white* means the wild-type *white*-cDNA of *Ceratitis capitata* (Zwiebel et al. 1995, Science 270, 2005, Loukeris et al. 1995, Science 270, 2002-2005). GFP is the S65T mutant of the *Aequorea victoria* gene encoding Green Fluorescent Protein (Heim et al. Nature 373:663-664, 1995). YP1P is the fat body promoter/enhancer of the gene encoding Yolk Protein 1 of *Drosophila melanogaster*. The *Xhol*I *Bam*HI fragment containing the YP1 promoter/enhancer (from bases -362 to +54, Genbank Accession Number X01524) was obtained by PCR from *Drosophila* (strain *yw*) DNA. The transposon is cloned in plasmid vector pTZ18-19 (Pharmacia).

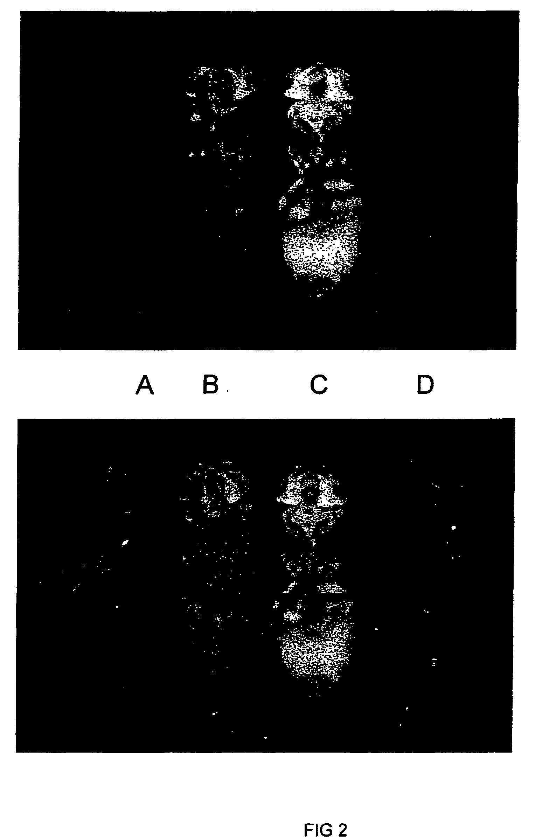

FIG. 2. Expression of GFP flies under the control of the *Drosophila* YP promoter in *Drosophila melanogaster*. (A) Homozygous transformed male; (B) heterozygous transformed female; (C) homozygous transformed female; (D) non-transformed female. The same field was photographed for epifluorescence (top) and for epifluorescence with faint incident light (bottom), to demonstrate non-fluorescing flies.

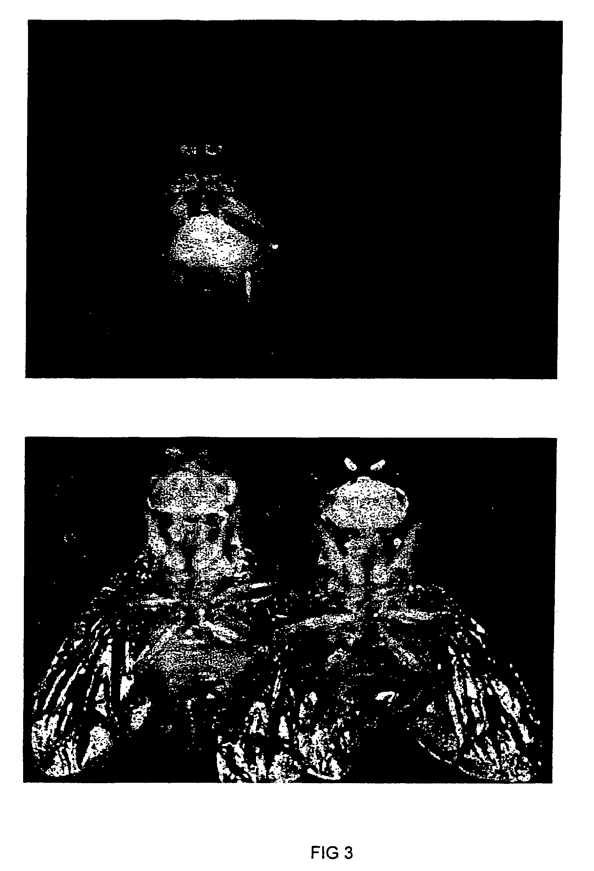

FIG. 3. Female-specific expression of GFP in Medflies under the control of the *Drosophila* YP promoter. A transformed female is shown on the left. A transformed male is shown on the right. Top: Epifluorescence. Bottom: Incident light.

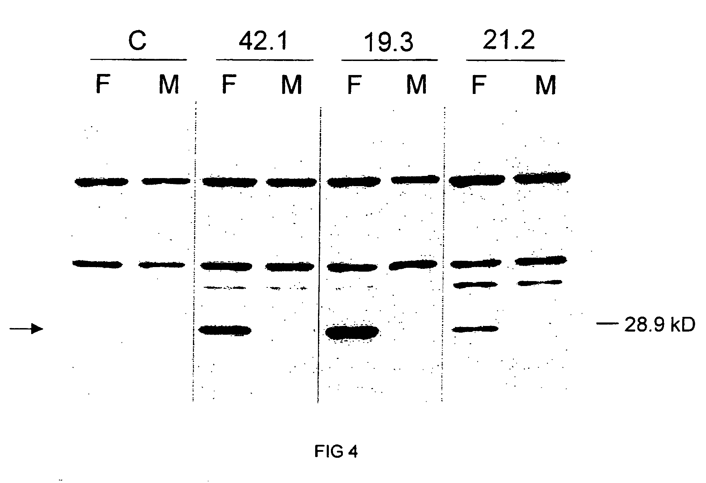

FIG. 4. Western-blot analysis of YP-promoter -directed expression of GFP in *Drosophila melanogaster* flies. The number identifying the transformed line is indicated at the top as C meaning a control, non-transformed fly, 42.1, 19.3, 21.2. F indicates female fly and M indicates male fly. Groups of ten flies (2-5 day old) were homogenized in 20 mM Tris.HCl pH 8.0 containing 10 mM EDTA, 5 mM β-mercaptoethanol and 30 mM NaCl. A supernatant from centrifugation with 14000xg was subjected to SDS-PAGE and blotted onto a nitrocellulose filter. An anti-GFP monoclonal antibody (CLONTECH Laboratories, Inc., Palo Alto, CA) and a commercial secondary antibody coupled to horseradish peroxidase were used for detection. The high molecular weight fragments are due to background.

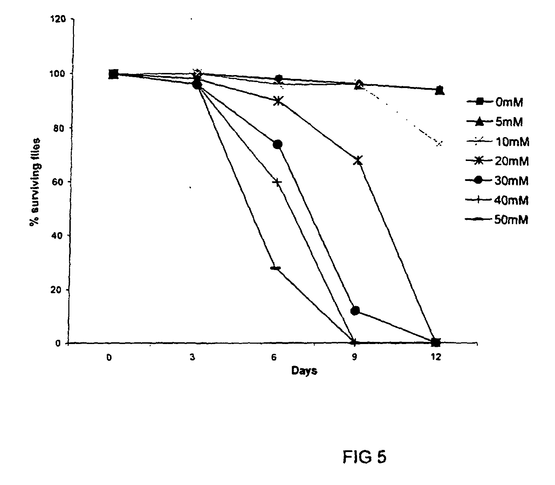

FIG. 5. Sensitivity of *D. melanogaster* (strain *yw*) to 5-FU. The *yw* strain is used routinely as recipient for generation of transgenic lines. Flies were reared in stadard *Drosophila* food supplemented with 5-FU at the indicated concentrations of 0 mM, 5 mM, 10 mM, 20 mM, 30 mM, 50 mM 5-FU. The x-axis represents time in days and the y-axis represents the number of flies. The data points represent the number of surviving flies.

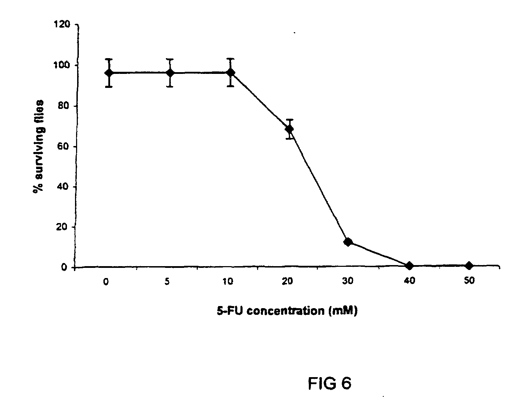

FIG. 6. Dose-dependent lethality of *D. melanogaster* to 5-FU. The x-axis represents 5-FU concentration in mM and the y-axis represents the number of flies. Strain yw flies that were used in the above described transformation experiment were reared in standard *Drosophila* food supplemented with 5-FU at the indicated concentrations and viable flies were determined after 9 days. Each data point represents the mean ± standard deviation of three separate experiments, each with 100 flies.

### DETAILED DESCRIPTION OF THE INVENTION

The present invention is applicable to control any target insect population, comprising any target insects, whether the population is homogenous or heterogeneous. For example, insects which are controlled using the present invention include but are not limited to *Bactrocera oleae* (olive fly) which affects olive crops; *Bactrocera orientalis* (oriental fruit fly) which affects many fruit crops; *Heliothis armigera* (cotton bollworm) which affects cotton; *Trichoplusa ni* (cabbage looper) which affects cabbages; *Manduca sexta* (tobacco hornworm) which affects tobacco; *Lobesia botrana* (grapevine moth) which affects grapes; *Anopheles gambiae* (mosquito) which carries malaria; *Aedes aegypti* (yellow fever mosquito) which causes yellow fever; *Glossina morsitans* (tse-tse fly) which carries African trypanosomiasis (sleeping sickness); *Simulium* sp. (black fly) which causes onchocerciacis (river blindness); *Phlebotomus* sp. (sand fly) which causes visceral leishmaniasis (kala-azar); *Musca domestica* (house fly) which spreads many bacterial infections. In preferred embodiment, the present invention is used in insect populations which include or consist of the Mediterranean fruitfly, *C. capitata* (Medfly).

### Pro-drug systems useful according to the present invention

Pro-drug-converting enzyme genes, also known as "suicide" genes have been used to subsequently activate cytotoxic drugs selectively in transfected tumor cells. Enzyme/pro-drug systems are known in the art. A pro-drug is a drug, often a potentially toxic drug, which is selectively activated, i.e. rendered toxic, by the action of an enzyme. Enzyme/pro-drug systems rely on the delivery of an enzyme to target cells or organisms, before administration of the pro-drug. Only target cells or organisms which express the enzyme will be affected by the pro-drug. One common enzyme/pro-drug system is the 5-fluorouracil/cytosine deaminase system, in which the non-toxic precursor 5-fluorocytosine (5-FC) is converted to the cytotoxic drug 5-fluorouracil (5-FU) by the action of cytosine deaminase (see Austin & Huber, (1993) Mol. Pharmacol. 43:380-387).

In one embodiment, enzymes that can be used for converting a pro-pesticide into an active pesticide, such as esterases, amidases and mixed-function oxidases which are also called P450, can be characterized and cloned from a variety of organisms, including eubacteria, archaebacteria and eukaria. A typical strategy for utilizing such an enzyme, e.g. metabolizing a specific pro-pesticide, would entail screening of organisms for presence of the enzymatic activity. A collection of organisms can be used for this purpose, such as a collection of cultured soil bacteria, a collection of insect tissues or cells or a collection of plant tissues or cells.

Screening can be performed, for example, by incubating the pro-pesticide with an extract from tissues or from bacteria. In the case of mixed function oxidases that are localized in microsomes from eukaryotic tissues, a microsomal fraction can be used as source of the activity. A microsomal fraction can be isolated by routine subcellular fractionation procedures. High speed supernatants of cell extracts can be used for other soluble enzymes. Determination of enzymatic activity can be performed by detecting the reaction product(s) by standard chemical analytical technologies well know to one skilled in art. For example, reaction product(s) can be separated using gas chromatography (GC). Mass spectrometry coupled to GC (GCMS) can be used to confirm the structure of the compounds.

Once the enzyme activity is determined, the gene encoding the activating enzyme can be cloned by using a variety of different cloning methods well know in art. For example, a nucleic acid library of the organism is generated using standard recombinant DNA technology. A genomic library for bacterial genes or a cDNA library for eukaryote genes can then be screened using standard methodology.

In one embodiment, hybridization with nucleic acid probes from a related gene, e.g. a cloned sterase or P450 gene from an evolutionarily related organism, is used. Alternatively, screening can be done by complementation. In the latter approach, an expression library, i.e. a library construed in a commercially available expression plasmid or viral vector, is used to transfect cells that do not normally express the enzymatic activity, and the clone containing the activating gene is isolated by screening individual colonies of the library for expression of enzymatic activity. A newly identified gene can then be cloned into vectors useful according to the invention, describe below.

Pro-drugs, as well as being inactive in organisms or cells that cannot convert them, may also have other advantages, such as improved lipid solubility and/or chemical stability.

Pro-insecticides or pro-drug toxins that have been designed to be convertible only in certain insects, are known in the art and have been prepared, for example, by selective derivatisation of the final toxin, especially in the case of organophosphates and carbamates. Examples include precursors of acetylcholinesterase inhibitors, such as parathion and profenofos, which are oxidised into active insecticides by enzymes of the cytochrome P-450 family. However, application of proinsecticides to insect populations only kills the insects which naturally express the enzyme. To render insect pests which do not naturally express the convening enzyme susceptible requires transfer of the gene encoding the converting enzyme into the host and a means of transfer of the gene in an environmentally acceptable manner within the target insect population

In one embodiment, the converting enzyme coding sequence encodes the esterase enzyme which converts DPX-JW062 into its active metabolite as disclosed by Wing et al. incorporated herein by reference (Wing et al., Arch Insect Biochem Physiol 37:91-103, 1998). The pro-insecticide is, in this aspect of the invention, DPX-JW062. DPX-JW062 is an oxadiazine compound bioconverted in lepdoptera into a potent toxin which blocks voltage-gated sodium channels. DPX-JW062 has low activity/toxicity in other organisms. Consequently, the expression of the lepdoptera converting enzyme in other insects will make the target insects susceptible to DPX-JW062 induced toxicity.

Alternatively, the coding sequence encodes an enzyme of the cytochrome P-450 family. Although most insects possess cytochrome P-450 family enzymes, the efficiency of bioconversion of proinsecticides converted by such enzymes may be increased by transforming the insect with a more efficient and/or overexpressed enzyme, allowing a decrease in the effective dose of the proinsecticide. Suitable proinsecticides in this aspect of the invention include organophosphates and carbamates. For example, the proinsecticide may be an acetylcholinesterase inhibitor such as parathion and profenofos. The cloning and expression of the orphid cytochrome P-450 enzymes responsible for the conversion of parathion and profenofos would allow a broadening of the susceptible range through the expression in otherwise resistant insect species.

Generally, genes useful in the practice of the invention, as well as vectors encoding them, may be prepared according to standard approaches used in molecular biology. See, for example, Sambrook et al., Molecular Cloning, A Laboratory Manual (1989) and Ausubel et al., Short Protocols in Molecular Biology (1999) 4th Ed, John Wiley & Sons, Inc.

A gene encoding cytosine deaminase (CD) is one example of the pro-drug-converting enzymes. The bacterial enzyme cytosine deaminase (CD ; EC 3.5.4.1) encoded by *cod A* is present in many bacteria and fungi but is absent in higher plants and animals. CD catalyzes the deamination of cytosine to uracil. Microorganisms that express the CD gene convert through a similar mechanism the non-toxic 5-fluorocytosine (5-FC) into the 5- fluorouracil (5-FU), a highly toxic metabolite that is lethal to the cell. Since higher organisms do not usually express cytosine deaminase they do not ordinarily metabolize cytosine to uracil. Moreover they do not deaminate 5-FC. The 5-FC is non-toxic to eukaryotes at concentrations that result in strong antimicrobial activity. However, 5-FU has potent cytotoxic effects on eukaryotic cells and is widely used as a chemotherapeutic agent in cancer treatment. The 5-FU is metabolized to 5-fluorouridine 5'-triphosphate and 5-fluoro-2'-deoxyuridine 5'-monophosphate, inhibiting both RNA and DNA synthesis and resulting in cell death. Thus, eukaryotic cells engineered to express CD should convert 5-FC to 5-FU and be selectively sensitive to 5-FC compared to native. unmodified cells. This was first demonstrated using human colorectal carcinoma cells transfected with a construct expressing bacterial CD. The transfected colorectal carcinoma cells are about 600-fold more sensitive to 5-FC than untransfected cells (Austin and Huber, 1993, Molec. Pharmacol. 43: 380-387).

According to a further embodiment, the pro-drug is p-hydroxyaniline mustard glucuronide, which is converted to the toxin p-hydroxyaniline mustard by the converting enzyme β-glucuronidase, which must be located in the extracellular space (Cheng et al., Biochem. Pharmacol. 58:325-328, 1999). Preferably, therefore, the coding sequence encodes E. coli β-glucuronidase.

### Female-specific regulatory regions according to the invention

A "regulatory region" which regulates a coding sequence includes one or more of promoters or one or more enhancers.

A "promoter" is herein defined as a nucleotide sequence which initiates transcription of a gene which it is operatively associated with. A "promoter" and/or "enhancer" is found on a nucleic acid fragment, which may be of any size, as long as it confers the stated activity when operatively associated with a gene or a coding sequence of interest. The fragment may be, e.g. 5 kilo base pairs (kb = 5000 base pairs); 1 kb; 500 nucleotides (nt); 200 nt; 100 nt; or even as small as 10-20 nt.

Yolk protein genes are highly conserved (Sappington and Raikhel, 1995, Insect Biochem Mol Biol 28, 277-300). Systems based on a number of *Drosophila* yolk protein genes have been characterized, (Ronaldson and Bownes, 1995, Genet Res 66:9-17) and used to effect female specific heterologous gene expression (see, Heinrich and Scott, 2000, PNAS 97:8229-8232; Thomas et al., 2000, Science 287:2474-2476). Yolk protein genes have also been characterized for the Medfly (Rina and Savakin, 1991, Genetics 127:769-780). The conserved nature of yolk protein genes indicates that regulatory elements driving heterologous genes in female insects can be isolated and characterized using standard genetic approach. Thus sex-specific promoters suitable for driving a coding sequence as set forth above are available in the art. In a preferred embodiment, vitellogenin genes encoding yolk proteins may be used as a source of suitable promoters. In the case where the target insects are Medfly (*C*. *capitata*), the VG1 and VG2 promoters may be used as disclosed in Rina and Savakis, Genetics 127:769-80, 1991.

*The Drosophila Yp-enhancer*. The Yolk protein (Yp) genes in *Drosophila melanogaster* follow a strict tissue- sex- and temporal-specific expression pattern, being transcribed only in adult female fat body and ovarian follicle cells. A fat body enhancer (FBE) located 196 bp upstream of the Yp1 cap site is sufficient to determine the sex-, stage- and fat body-specific expression of both yp1 and yp2 genes as described by Garabedian et al., Cell 45:859-867, 1985. More recently, a simple enhancer (33 bp) within the FBE has been postulated to be involved in the regulation of the yp gene expression. This female- and fat body-specific enhancer consists of two enhancer elements (o and r). One element (22 bp) contains two protein binding sites, the dsx A and an overlapping bzip1, that binds the DmC/EBP (*slbo*) protein, a member of the bZIP family of transcriptional activators. The other element is an 11 bp binding site for an unknown protein (An and Wensink, 1995, EMBO J. 14:1221-1230).

### Vectors and transformation according to the invention

The transformation of target insects may be performed using any suitable technique for preparing transgenic insects. Currently, there are several art recognized techniques such as DNA transfection using transgesons, the use of viral vectors, and the use of episomal vectors which are transmitted through germ cells. Preferably, the transformation is carried out using a suitable vector. The invention thus provides a vector which is capable of transforming a target insect cell, which vector comprises a gene comprising a coding sequence encoding one constituent of an enzyme/pro-drug system and a promoter capable of driving the coding sequence in target insects in a sex-specific manner.

Preferably, the vector is a transposon or transposable element. The invention accordingly provides a method as set forth above, wherein the transformation step comprises the steps of:
(a) providing transposon either as a transposable element which encodes a transposase protein which is active in the target insects; an RNA encoding a transposase or purified transposase protein.
(b) modifying the transposable element by inserting reglatory elements and the gene encoding the activity enzyme; and
(c) transforming the target insects with the modified transposable element in the presence of transposase activity..

Transposons are genetic elements which are capable of "jumping" or transposing from one position to another within the genome of a species. They are widely distributed amongst animals, including insects. Transposons are active within their host species due to the activity of a transposase protein encoded by the elements themselves. Advances in the understanding of the mechanisms of transposition have resulted in the development of genetic tools based on transposons which can be used for gene transfer.

Any transposable element active in the desired target insect may be used. Preferably, however, the transposable element is selected from the Tet/Mariner superfamily of transposons consisting essentially of Minos, mariner, Hermes and piggyBac.

Minos is a transposable element which is active in Medfly. It is described in US patent 5,840,865. The use of Minos to transform insects is described in the foregoing US patent.

Mariner is a transposon originally isolated from *Drosophila*, but since discovered in several invertebrate and vertebrate species. The use of mariner to transform organisms is described in International patent application WO99/09817.

Hermes is derived from the common housefly. Its use in creating transgenic insects is described in US patent 5,614,398.

PiggyBac is a transposon derived from the baculovirus host *Trichplusia ni*. Its use for germ-line transformation of Medfly has been described by Handler et al., (1998) PNAS (USA) 95:7520-5.

According to the present invention, insects are generated which can be released into wild-type populations of insects. The transgenic insects of the invention will consequently interbreed with the wild-type populations to produce target insects which are susceptible to a proinsecticide. The invention thus relates to an insect of a given sex, which insect has been transformed with a gene comprising a coding sequence encoding one constituent of an enzyme/pro-drug system and a promoter capable of driving the coding sequence substantially only in insects of the opposite sex. Preferably, the insect is a male insect. Preferably the insect can be mass reared. More preferably, the insect is a Medfly.

### Designing pro-pesticides from pre-existing pesticides according to the invention

To create non-toxic pro-insecticides, currently available insecticides can be rendered inactive by derivatizing a chemical group that is required for biological activity. Such groups include, but are not limited to, amino or imino groups. Derivatization can be performed by, for example, methylation or acetylation.

Metabolic activation is an enzymatic process in the target species that converts the pesticide to a biologically more active structure. Thus, weak inhibitors or noninhibitors may be accidentally changed into products of lethal toxicity by systems designed to degrade xenobiotic compounds (p. 129 in Insecticide Mode of Action ed. Joel R. Coats, Academic Press, 1982).

In the general field of phosphate insecticides, the most common activation process mediated by mixed-function oxidase is the P → S to P → O conversion, e.g. parathion to paraoxon. (Nakatsogawa and Morelli, 1976; Ero, 1974; Fukuto, 1978).

In one embodiment of the invention, acephate-methamidophos propesticide-pesticide system can be used. Methamidophos (O, S-dimethyl phosphoramidothicate, C₂H₈NO₂PS) was introduced in 1969 by Chevron Chemical and Bayer. It is a highly active, systemic, residual organophosphate insecticide/acaricide with contact and stomach action. Methamidophos is a potent acetylcholinesterase (AchE) inhibitor. It is effective against chewing and sucking insects and is used to control aphids, flea beetles, worms, whiteflies, thrips, cabbage loopers, Colorado potato beetles, potato tubeworms, armyworms, mites, leafhoppers, and many others. Toxicity of methamidophos has been determined as acute oral LD50 values of 21 and 16 mg/kg body weight for male and female rats respectively.

Acephate is a N-acetyl derivative of methamidophos (O, S-dimethyl acetylphosphoramidothicate, C₉H₁₀NO₃PS) which was introduced by Chevron Chemical as a second-generation improvement of methamidophos. It is used for control of a wide range of biting and sucking insects, especially aphids, including resistant species, in fruit, vegetables (e.g. potatoes and sugar beets), vine, and hop cultivation and in horticulture (e.g. on roses and chrysanthemums grown outdoors). Acephate and its primary metabolite, methamidophos, are toxic to *Heliothis* spp. that are considered resistant to other organophosphate insecticides. Toxicity of Acephate has been determined as acute oral LD50 Value A 945 and 866 mg/kg for male and female rats respectively (45 to 54-fold less toxic than its metabolite methamidophos).

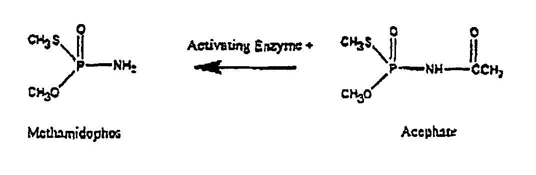

Acephate itself is not an AchE inhibitor; its conversion to methamidophos, which is an inhibitor, requires an enzyme-mediated cleavage of the carbonyl-N bond. Nearly all living systems contain amidases that cleave simple aliphatic amides. However, acephate is not a simple amide and some nonspecific amidases may find it a poor substrate. Methamidophos has been observed as the principle metabolite of acephate in bean, cabbage and tomato seedlings (Tucker, 1972, Report, Chevron Chemical Company, Richmont, California). The cleavage of the carbonyl-N bond is not a nonspecific process, as is indicated by findings that activity of the activating amidases is highly sensitive to variations in the acephate structure.

There appears to be a direct relation of the activation reaction and toxicity in target insects. In the tobacco budworm *Heliothis virescens* acephate and methamidophos show comparable activity. A small amount of methamidophos was observed in the budworm larvae within 2 hours of a topical acephate application. However, in the adult boll weevil *Anthonomus grandis* Boheman methamidophos is 75-fold more toxic than acephate despite rapid absorbsion of both compounds. No metabolism of acephate to methamidophos was observed in the bolI weevil, a fact consistent with its low toxicity (Bull, 1979, J. Agric. Food Chem. 27:268).

Many living organisms possess one or more amidases that cleave a significant fraction of applied acephate to methamidophos. Animals such as the rat (Tucker, 1976) and the female white mouse (Kao and Fukuto, 1977) also convert a significant amount of acephate or its analogs to methamidophos. In their study on the propionyl and hexanoyl analogs, Kao and Fukuto were able to relate the toxicity directly to the amount of activation. This strongly suggests that unconverted acephate is essentially nontoxic.

Thus the combination of a gene encoding an amidan enzyme capable of converting acephate to its active form methamidophos will render an insect susceptible to applications of the previously non-toxic acephate.

It has also been reported that the toxicity of acephate analogs to the house fly is consistent with the amount of metabolically formed methamidophos; the propionyl analog generates substantially more methamidophos in this specis and is 35-fold more toxic (Kao and Fukuto, 1977, *Pestc. Biochem. Physiol.* 7:83).

In another embodiment of the invention, Imidacloprid (1-[8-chloro-3-pyridinyl)methyl]-N-nitro-2-imidazolidinimine, C₉H₁₀CIN₅O₂) and N-Me-imidacloprid can be used as a pro-pesticide system.

Imidacloprid is a systemic, chloro-nicotinyl insecticide for the control of sucking insects including rice hoppers, aphids, thrips, whiteflies, termites, turf insects, soil insects and some beetles. It is most commonly used on rice, cereal, maize, potatoes, vegetables, sugar beets, fruit, cotton, hops and turf, and is especially systemic when used as a seed or soil treatment. The chemical works by interfering with the nicotinergic acetylcholine receptor (nAcR) which is more abundant in insects than in warm-blooded animals. It is effective on contact and via stomach action. (Kidd, H. and James, D.R., Eds. The Agrochemicals Handbook, Third Edition. Royal Society of Chemistry Information Services, Cambridge, UK, 1991 (As Updated). 10-2). Toxicity for imidacloprid has been determined as acute oral LD50 in rats is 450 mg/kg body weight.

N-Me-imidacloprid is an imidacloprid pro-insecticide. Introduction of a methyl group at the 3 position of the imidazole ring imidacloprid decreases binding affinity to nAcR 100-fold. However, N-Me-imidacloprid is as potent as imidacloprid in the housefly. N-Me-imidacloprid can be convened to imidacloprid by an in vitro mixed function oxidase system (Yamamoto et al.. 1998, Arch. Insect Biochem. Physiol. 37:24). The compound is not commercially available. Currently, no toxicity data are available for N-Me-imidacloprid.

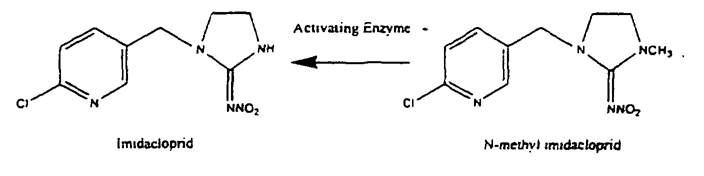

The conversion of N-Me-imidacloprid to imidacloprid in the housefy indicates the presence of enzymes capable of effecting pro-pesticide conversion. *See Magee (1982) in Structure-Act In Vivo relationships in phosphoramidates, Chp 5*, *Insecticide made action Academic Press*.

The resulting derivative (pro-pesticide) can be metabolized to the active pesticide by enzymes or "activating enzymes" that occur or are transfected into target insects. Several activating enzymes can be used including but not limited to oxidases (P450), esterases and amidases.

In summary a gene encoding an activating enzyme that converts a pro-pesticide into an active pesticide is introduced into the genome of pest species that do not normally produce the activating enzyme or have low levels of activating enzyme. The sex specific expression of the activating enzyme in the target pest population enhances the spread of the gene in the local pest population when combined with the application of the pro-pesticide systemically or non-systemically. In the preferred embodiment this will result in the selective elimination of the female pest population. Recombinant surviving males will then breed driving the activation gene through the local population resulting after cycles of application of proinsecticide in the reduction or elimination of female pests and hence reduction or elimination of a viable local pest population.

Designing a pro-pesticide which is based on a registered pesticide such as imidacloprid has an important economical advantage over introducing a novel pro-pesticide: The known toxicology of the pesticide is utilized instead of establishing the extensive toxicology analysis of a novel compound which decreases time to market and costs for toxicology analysis.

### EXAMPLES

### Example 1. Preparation of a construct encoding E. coli cytosine deaminase

Using standard methods, a genetic construct is prepared bearing the C. capitata VG1 promoter operably linked to a cDNA encoding E. coli cytosine deaminase. The cytosine deaminase gene, when introduced into insect cells, confers sensitivity to the pro-drug 5-FC.

The construct is inserted into the Minos transposon using conventional techniques and, in addition, those described in US 5, 840, 865. The modified transposon is then used to transform Medfly according to standard transgenesis procedures.

Transgenic flies carrying the modified transposon are bred to create transgenic strains that express the cytosine deaminase enzyme. Characterisation for cytosine deaminase activity shows that the enzyme is expressed at a high level selectively in females. Moreover, cytosine deaminase expression is correlated with susceptibility to the pro-drug 5-FC administered in food to the flies.

A highly susceptible strain is bred and male flies isolated. Introduction of male flies into a population of non-transgenic flies results in rapid transfer of the transgene. Female flies which are born inheriting the transgene are susceptible to 5-FC, whilst male flies remain resistant.

### Example 2. Preparation of the Yp-CDASE construct

Two specific oligonucleotides which correspond to the published sequence of the CD gene (Austin and Huber, 1993, Molec. Pharmacol. 43:380-387) were used in polymerase chain reaction (PCR). The first oligonucleotide was used to introduce a more closely matching Kozak consensus start codon "ATG" in the place of "GTC" in the published sequence. Oligonucleotide 5'- CGGGATCCACCATGTCGAATAACGCTTTAC-3' corresponds to the 5' end of the CD sequence but changes the start codon from GTG to ATG, more closely matches the Kozak consensus sequence (Kozak, 1986, Cell 44: 283-292) The second oligonucleotide introduced BamHI-restriction site starting 9 bp upstream from the ATG. Oligonucleotide 5'-GCTCTAGATCAACGTTTGTAATCGATGGC-3' corresponds to the 3' end of the CD sequence and contains an *Xba I* restriction site. A 1.3 kb DNA fragment of the modified CD gene was amplified by PCR using template DNA from an XL1 blue *E. coli* strain. The PCR product was cloned into a pGEM-T Easy vector (Promega, Madison, WI).

As a first step in the construction of a female-specific CD expression vector, a *Not I* fragment containing the CD gene was cloned into pCasper-hs. Vector pCasper-hs (Thummel and Pirrotta, 1992, Technical Notes. D.I.S. 71; 150) is a P element based transformation vector containing a Hsp70 promoter/terminator construct that can be used for expressing open reading frames under heat shock control. In the second step, the resulting plasmid, pCasper-hs-CD, was cut with *Xho*I/*Bgll I* and a *XhoI*I/*Bam*HI fragment containing the YP1 promoter/enhancer was used to replace the Hsp70 promoter. The resulting plasmid vector pCasper-YP-CD was used to transform *Drosophila*.

### Example 3. Female-specific expression of green fluorescent protein (GFP) using Drosophila Yp-enhancer

Ten transgenic *Drosophila* lines were generated using Minos-mediated germ line transformation, each carrying a single copy of a transposon (MiYP/GFP-w) that contains the *Aequorea victoria* gene for the GFP driven by the *Drosophila melanogaster* Yp1 fat body promoter/emhancer (FIG. 1). In all lines, GFP fluorescence is detectable in the fat body of adult females, is dose-dependent, but it is undetectable in adult males (FIG. 2). 15 medfly transgenic lines have been generated using the same transposon. FIG. 2 shows female-specific expression of GFP driven by the *Drosophila* YP promoter in one of these medfly lines.

Sex-specific expression of GFP protein in some of these transgenic lines has also been documented by Western blot analysis using a commercially available monoclonal anti-GFP antibody (FIG. 4).

### Example 3. Transformation of Drosophila.

Transformation was performed using previously described methodology (Rubin and Spradling, 1982, Science 218, 348-353). Transformants were detected by screening the G1 progeny of the injected flies for expression of the dominant *white* marker present in pCasper-YP-CD.

### Example 4. Detection of female-specific expression of CDASE by detection of CD enzymatic activity

CD enzyme activity was determined both in transformed flies, females and males, and in a yw-strain which lacks CD expression. The ability of flies to deaminate 5-FC to 5-FU was assayed spectrophotometrically as described previously (Austin and Huber1993, Molec. Pharmacol. 43: 380-387). Briefly, frozen flies were homogenized in homogenization buffer (100mM Tris-Cl, pH 7.4, 1mM ethylenediaminetetraacetic acid [EDTA], 5mM β-mercaptoethanol and 0.5 mM PMSF) using an Ultra-Turrex homogenizer. The homogenate was centrifuged at 14,000g for 30 min and the supernatant was used for the assay. CD activity was measured in a1-ml assay mixture containing 50 mM Tris-Cl, pH 7.3, 50 µl of supernatant and 0.5 mM cytosine or 5-FC. Decreases in absorbance at 285 nm were measured over time. Product was estimated by using a molar extinction coefficient of 1.038 x 10⁻³ M⁻¹cm⁻¹ for cytosine to uracil and 1.96 mM⁻¹ cm⁻¹ for 5-FC to 5-FU.

### Example 5. Detection of female-specific expression of CDASE by detection of CD protein

Whole fly extracts that were prepared for CD enzymatic assays described above, were also analyzed on SDS-PAGE. A fragment migrating with an apparent molecular weight of approximately 52,000 was detected in extracts prepared from transformed female flies; this fragment was not detectable in non-transformed flies or in transformed male flies. The identity of the 52 kD band was confirmed with Western blot analysis using a polyclonal antibody for CD.

### Example 6. Determination of the female toxicity by feeding variable concentrations of active compound

To evaluate the effects of 5-FU on *a D. melanogaster* yw strain, flies were raised first on medium containing 5-FU. Groups of 25 flies were cultured on food containing various concentrations of the drug. Four vials of each concentration of the drug were used for each experiment and three experiments were performed. The surviving flies per vial were counted over a period of time. FIG. 5. shows survival rates over time for several concentrations of the drug; FIG. 6. shows dose-response for survival at nine days. 5-FU treatment caused efficient killing of flies in a concentration range 30-50 mM within 9 days. No eggs were laid on food containing more than 20 mM of 5-FU.

The effects of the pro-drug 5-FC on non-transformed adult flies were determined in a similar way. Pro-drug treatment of non-transformed flies resulted in substantially no reduction of fly viability.

### Example 7. Determination of female toxicity by using variable concentrations of pro-drug

The effect of the pro-drug 5-FC on CD transformed flies was determined by raising the flies on food containing various concentrations of 5-FC. Female CD files showed a substantial reduction in viability with increasing 5-FC when compared with male CD files.

### OTHER EMBODIMENTS

Other embodiments will be evident to those of skill in the art. It should be understood that the foregoing detailed description is provided for clarity only and is merely exemplary.

## Claims

1. A method for controlling a population of target insects, comprising:
(i) providing a gene comprising a coding sequence encoding one constituent of an enzyme/pro-pesticide system and a regulatory region operatively linked to the coding sequence in the target insects in a sex specific manner;
(ii) transforming at least part of the population of target insects with the gene, and allowing the transposable elements to spread within the target insect population; and
(iii) administering to the population of target insects the remaining constituent(s) of the enzyme/pro-pesticide system, wherein the enzyme is capable of catalysing the conversion of the pro-pesticide to a pesticide.

2. A method according to claim 1, wherein the promoter is active to express the coding sequence predominantly in male target insects.

3. A method according to claim 1 or claim 2, wherein the regulatory region is active to express the coding sequence predominantly in female target insects.

4. A method according to any preceding claim, wherein the coding sequence encodes an enzyme which converts a pro-pesticide into its active metabolite.

5. A method according to any preceding claims, wherein the coding sequence encodes an amidase enzyme which converts a pro-pesticide into its active metabolite.

6. A method according to any preceding claim, wherein the coding sequence encodes an mixed functional oxidase/cytochrome P450 which converts a pro-pesticide into its active metabolite.

7. A method according to any preceding claim, wherein the coding sequence encodes an esterase enzyme which converts a pro-pesticide into its active metabolite.

8. A method according to any of the preceding claims wherein the coding sequence encodes cytosine deaminase.

9. A method according to any of the preceding claims wherein the coding sequence encodes b-glucuronidase.

10. A method according to claim 4 wherein the pro-pesticide comprises an organophosphate, phosphoramidate, neonicotinoid, or oxadiazine derivative.

11. A method according to claim 5, wherein the pro-pesticide/activation enzyme comprises:
(i) acephate or an analogue of acephate;
(ii) a amidase capable of converting acephate or an analogue of acephate to its active metabolite methamidophos.

12. A method according to claim 6 wherein the pro-pesticide/activation enzyme comprises:
(i) N-Me-imidacloprid or an analogue of N-Me-imidacloprid;
(ii) a mixed functinal oxidase/cytochrome P450 capable of converting N-Me-imidacloprid or an analogue of N-Me-imidacloprid to its active metabolite imidacloprid.

13. A method according to claim 7, wherein pro-pesticide activation enzyme comprises:
(i) DPX-JWO62 or an analogue of DPX-JW062;
(ii) an esterase capable of converting DPX-JW062 or an analogue of DPX-JW062 to its active metabolite.

14. A method according to claim 8 wherein the pro-pesticide/activation enzyme further comprises 5-FC.

15. A vector which is capable of transforming a target insect cell, which vector comprises a gene comprising a coding sequence encoding one constituent of an enzyme/pro-pesticide system and a regulatory region operatively linked to the coding sequence in target insects in a sex-specific manner, wherein the enzyme is capable of catalysing the conversion of the pro-pesticide to a pesticide.

16. A vector which is capable of transforming a target insect cell, which vector comprises a gene comprising a coding sequence encoding one constituent of an enzyme/pro-pesticide system and a promoter capable of driving the coding sequence in target insects in a sex-specific manner, wherein the enzyme is capable of catalysing the conversion of the pro-pesticide to a pesticide.

17. A vector according to claims 15 and 16 which is a transposon.

18. A transposon according to claim 17 which is Minos.

19. A method according to any preceding claim wherein the target insect is *Ceratitis capitata*.

20. A method according to claim 16, wherein the promoter is a *Ceratitis capitata* yolk protein gene promoter.

21. An insect of a given sex, which insect has been transformed with a gene comprising a coding sequence encoding one constituent of an enzyme/pro-pesticide system and a promoter capable of driving the coding sequence substantially only in insects of the opposite sex, wherein the enzyme is capable of catalysing the conversion of the pro-pesticide to a pesticide.

22. An insect according to claim 21 which is a male insect.

23. A male insect selected according to claims 21 and 22 and subsequently irradiated for applications in sterile insect technique.

24. An insect according to claim 23 which is *Ceratitis capitata*.

## Patentansprüche

1. Verfahren zum Steuern einer Population von Zielinsekten mit:
(i) Bereitstellen eines Gens, welches eine codierende Sequenz, die einen Bestandteil eines Enzym/Pro-Pestizid-Systems codiert, und einen regulatorischen Bereich, welcher in den Zielinsekten in einer geschlechtsspezifischen Art und Weise funktionsfähig mit der codierenden Sequenz verknüpft ist, umfaßt,
(ii) Transformieren von wenigstens einem Teil der Population von Zielinsekten mit dem Gen und Zulassen, daß sich die transponierbaren Elemente innerhalb der Zielinsektenpopulation verbreiten, und
(iii) Verabreichen des bzw. der verbleibenden Bestandteils bzw. Bestandteile des Enzym/Pro-Pestizid-Systems an die Population von Zielinsekten, wobei das Enzym in der Lage ist, die Umwandlung des Pro-Pestizids in ein Pestizid zu katalysieren.

2. Verfahren nach Anspruch 1, wobei der Promotor dahingehend aktiv ist, daß er die codierende Sequenz überwiegend in männlichen Zielinsekten exprimiert.

3. Verfahren nach Anspruch 1 oder Anspruch 2, wobei der regulatorische Bereich dahingehend aktiv ist, daß er die codierende Sequenz überwiegend in weiblichen Zielinsekten exprimiert.

4. Verfahren nach einem der vorangegangenen Ansprüche, wobei die codierende Sequenz ein Enzym codiert, welches ein Pro-Pestizid in dessen aktiven Metaboliten umwandelt.

5. Verfahren nach einem der vorangegangenen Ansprüche, wobei die codierende Sequenz ein Amidaseenzym codiert, welches ein Pro-Pestizid in dessen aktiven Metaboliten umwandelt.

6. Verfahren nach einem der vorangegangenen Ansprüche, wobei die codierende Sequenz ein gemischt-funktionales Oxidase/Cytochrom P450 codiert, welches ein Pro-Pestizid in dessen aktiven Metaboliten umwandelt.

7. Verfahren nach einem der vorangegangenen Ansprüche, wobei die codierende Sequenz ein Esteraseenzym codiert, welches ein Pro-Pestizid in dessen aktiven Metaboliten umwandelt.

8. Verfahren nach einem der vorangegangenen Ansprüche, wobei die codierende Sequenz eine Cytosindesaminase codiert.

9. Verfahren nach einem der vorangegangenen Ansprüche, wobei die codierende Sequenz b-Glucuronidase codiert.

10. Verfahren nach Anspruch 4, wobei das Pro-Pestizid ein Organophosphat-, ein Phosphoramidat-, ein Neonikotinoid- oder ein Oxadiazin-Derivat umfaßt.

11. Verfahren nach Anspruch 5, wobei das Pro-Pestizid/Aktivierungsenzym folgendes umfaßt:
(i) Acephate oder ein Analoges von Acephate,
(ii) eine Amidase, die in der Lage ist, Acephate oder ein Analoges von Acephate in dessen aktiven Metaboliten Methamidophos umzuwandeln.

12. Verfahren nach Anspruch 6, wobei das Pro-Pestizid/Aktivierungsenzym folgendes umfaßt:
(i) N-Me-Imidacloprid oder ein Analoges von N-Me-Imidacloprid,
(ii) ein gemischt-funktionales Oxidase/Cytochrom P450, welches in der Lage ist, N-Me-Imidacloprid oder ein Analoges von N-Me-Imidacloprid in dessen aktiven Metaboliten Imidacloprid umzuwandeln.

13. Verfahren nach Anspruch 7, wobei das Pro-Pestizid/Aktivierungsenzym folgendes umfaßt:
(i) DPX-JW062 oder ein Analoges von DPX-JW062,
(ii) eine Esterase, die in der Lage ist, DPX-JW062 oder ein Analoges von DPX-JW062 in dessen aktiven Metaboliten umzuwandeln.

14. Verfahren nach Anspruch 8, wobei das Pro-Pestizid/Aktivierungsenzym weiterhin 5-FC umfaßt.

15. Vektor, welcher in der Lage ist, eine Zielinsektenzelle zu transformieren, wobei der Vektor ein Gen mit einer codierenden Sequenz, die einen Bestandteil eines Enzym/Pro-Pestizid-Systems codiert, und einen regulatorischen Bereich, der mit der codierenden Sequenz in Zielinsekten in einer geschlechtsspezifischen Art und Weise funktionsfähig verknüpft ist, umfaßt, wobei das Enzym in der Lage ist, die Umwandlung des Pro-Pestizids in ein Pestizid zu katalysieren.

16. Vektor, welcher in der Lage ist, eine Zielinsektenzelle zu transformieren, wobei der Vektor ein Gen mit einer codierenden Sequenz, die einen Bestandteil eines Enzym/Pro-Pestizid-Systems codiert, und einen Promotor, der in der Lage ist, die codierende Sequenz in Zielinsekten in einer geschlechtsspezifischen Art und Weise zu steuern, umfaßt, wobei das Enzym in der Lage ist, die Umwandlung des Pro-Pestizids in ein Pestizid zu katalysieren.

17. Vektor nach Anspruch 15 oder 16, welcher ein Transposon ist.

18. Transposon nach Anspruch 17, welches Minos ist.

19. Verfahren nach einem der vorangegangenen Ansprüche, wobei das Zielinsekt *Ceratitis capitata* ist.

20. Verfahren nach Anspruch 16, wobei der Promotor ein *Ceratitis capitata*-Eidottergenpromotor ist.

21. Insekt mit einem vorgegebenen Geschlecht, wobei das Insekt mit einem Gen transformiert wurde, welches eine codierende Sequenz, die einen Bestandteil eines Enzym/Pro-Pestizid-Systems codiert, und einen Promotor, der in der Lage ist, die codierende Sequenz im wesentlichen nur in Insekten des entgegengesetzten Geschlechts zu steuern, umfaßt, wobei das Enzym in der Lage ist, die Umwandlung des Pro-Pestizids in ein Pestizid zu katalysieren.

22. Insekt nach Anspruch 21, welches ein männliches Insekt ist.

23. Männliches Insekt, welches nach Anspruch 21 oder 22 ausgewählt und anschließend für Anwendungen in einer Technik mit sterilen Insekten bestrahlt wurde.

24. Insekt nach Anspruch 23, welches *Ceratitis capitata* ist.

## Revendications

1. Procédé pour lutter contre une population d'insectes cibles, comprenant les étapes consistant :
(i) à fournir un gène comprenant une séquence codante codant pour un constituant d'un système enzyme/pro-pesticide et une région régulatrice liée de manière fonctionnelle à la séquence codante chez des insectes cibles d'une manière spécifique du sexe ;
(ii) à transformer au moins une partie de la population d'insectes cibles avec le gène, et à laisser les éléments transposables se disséminer dans la population d'insectes cibles ; et
(iii)à administrer à la population d'insectes cibles le ou les constituants restants du système enzyme/pro-pesticide, l'enzyme étant capable de catalyser la conversion du pro-pesticide en un pesticide.

2. Procédé suivant la revendication 1, dans lequel le promoteur est actif pour exprimer la séquence codante principalement chez les insectes cibles mâles.

3. Procédé suivant la revendication 1 ou la revendication 2, dans lequel la région régulatrice est active pour exprimer la séquence codante de manière prédominante chez les insectes cibles femelles.

4. Procédé suivant l'une quelconque des revendications précédentes, dans lequel la séquence codante code pour une enzyme qui convertit un pro-pesticide en son métabolite actif.

5. Procédé suivant l'une quelconque des revendications précédentes, dans lequel la séquence codante code pour une enzyme amidase qui convertit un pro-pesticide en son métabolite actif.

6. Procédé suivant l'une quelconque des revendications précédentes, dans lequel la séquence codante code pour une enzyme mixte fonctionnelle oxydase/cytochrome P450 qui convertit un pro-pesticide en son métabolite actif.

7. Procédé suivant l'une quelconque des revendications précédentes, dans lequel la séquence codante code pour une enzyme estérase qui convertit un pro-pesticide en son métabolite actif.

8. Procédé suivant l'une quelconque des revendications précédentes, dans lequel la séquence codante code pour la cytosine-désaminase.

9. Procédé suivant l'une quelconque des revendications précédentes, dans lequel la séquence codante code pour la β-glucuronidase.

10. Procédé suivant la revendication 4, dans lequel le pro-pesticide comprend un organophosphate, un phosphoramidate, un néonicotinoide ou un dérivé d'oxadiazine.

11. Procédé suivant la revendication 5, dans lequel le pro-pesticide/enzyme activatrice comprend :
(i) l'acéphale ou un analogue d'acéphate ;
(ii) une amidase capable de convertir l'acéphate ou un analogue d'acéphate en son métabolite actif méthamidophos.

12. Procédé suivant la revendication 6, dans lequel le pro-pesticide/enzyme activatrice comprend :
(i) le N-Me-imidaclopride ou un analogue de N-Me-imidaclopride ;
(ii) une enzyme mixte fonctionnelle oxydase/cytochrome P450 capable de convertir le N-Me-imidaclopride ou un analogue de N-Me-imidaclopride en son métabolite actif imidaclopride.

13. Procédé suivant la revendication 7, dans lequel l'enzyme d'activation du pro-pesticide comprend :
(i) l'enzyme DPX-JW062 ou un analogue de DPX-JW062 ;
(ii) une estérase capable de convertir la DPX-JW062 ou un analogue de DPX-JW062 en son métabolite actif.

14. Procédé suivant la revendication 8, dans lequel le pro-pesticide/enzyme activatrice comprend en outre du 5-FC.

15. Vecteur qui est capable de transformer une cellule d'insecte cible, vecteur qui comprend un gène comprenant une séquence codante codant pour un constituant d'un système enzyme/pro-pesticide et une région régulatrice liée de manière fonctionnelle à la séquence codante chez des insectes cibles d'une manière spécifique du sexe ; l'enzyme étant capable de catalyser la conversion du pro-pesticide en un pesticide.

16. Vecteur qui est capable de transformer une cellule d'insecte cible, vecteur qui comprend un gène comprenant une séquence codante codant pour un constituant d'un système enzyme/pro-pesticide et un promoteur capable de commander la séquence codante chez les insectes cibles d'une manière spécifique du sexe, l'enzyme étant capable de catalyser la conversion du pro-pesticide en un pesticide.

17. Vecteur suivant les revendications 15 et 16, qui est un transposon.

18. Transposon suivant la revendication 17, qui est le transposon Minos.

19. Procédé suivant l'une quelconque des revendications précédentes, dans lequel l'insecte cible est *Ceratitis capitata*.

20. Procédé suivant la revendication 16, dans lequel le promoteur est un promoteur de gène de protéine de vitellus de *Ceratitis capitata*.

21. Insecte d'un sexe donné, insecte qui a été transformé avec un gène comprenant une séquence codante codant pour un constituant d'un système enzyme/pro-pesticide et un promoteur capable de commander la séquence codante pratiquement seulement chez les insectes du sexe opposé, l'enzyme étant capable de catalyser la conversion du pro-pesticide en un pesticide.

22. Insecte suivant la revendication 21, qui est un insecte mâle.

23. Insecte mâle choisi suivant les revendications 21 et 22 et ensuite irradié pour des applications dans une technique d'obtention d'insectes stériles.

24. Insecte suivant la revendication 23, qui est *Ceratitis capitata*.
